# EUROPEAN PATENT SPECIFICATION

(11) **EP 2 941 203 B1**
(45) Date of publication and mention of the grant of the patent: **18.12.2019**
(21) Application number: 13711506.9
(22) Date of filing: 06.03.2013
(51) Int. Cl.: A61B 17/12

(54) **RAPID EXCHANGE TEMPORARY BLOOD FLOW CESSATION DEVICE FOR LARGE BORE CLOSURE**
SCHNELL AUSTAUSCHBARE TEMPORÄRE BLUTFLUSSSTOPPVORRICHTUNG FÜR GROSSEN VERSCHLUSS
DISPOSITIF DE CESSATION DE CIRCULATION SANGUINE TEMPORAIRE À ÉCHANGE RAPIDE POUR FERMETURE DE GRANDS ORIFICES

(30) Priority: 04.01.2013 US 201361749176 P
(43) Date of publication of application: 11.11.2015
(73) Proprietor: St. Jude Medical Puerto Rico LLC, Cagus 00726 (PR)
(72) Inventor: TEGELS, Zachary, J., Minneapolis, MN 55419 (US)
(74) Representative: Evens, Paul Jonathan
(86) International application number: PCT/US2013/029237
(87) International publication number: WO 2014/107178

(56) References cited:
- EP-A1- 1 479 409
- WO-A1-00/07657
- WO-A1-2012/162651
- US-A- 5 419 765
- US-A- 6 048 357
- US-A- 6 136 010
- US-A1- 2003 167 038
- US-A1- 2006 064 074
- US-A1- 2007 016 133
- US-A1- 2008 077 085
- US-A1- 2008 171 979
- US-A1- 2011 282 195
- US-A1- 2013 006 174
- US-B1- 6 217 547
- US-B2- 6 893 416

## Description

### CROSS-REFERENCE TO RELATED APPLICATION

This application claims the benefit of the filing date of United States Provisional Application No. 61/749,176, filed January 4, 2013, and entitled RAPID EXCHANGE TEMPORARY BLOOD FLOW CESSATION DEVICE FOR LARGE BORE CLOSURE.

### TECHNICAL FIELD

The present disclosure relates to closure devices, and more specifically relates to closure devices that temporarily occlude blood flow through a vessel as part of a method of sealing a puncture in the vessel.

### BACKGROUND

Various medical procedures, particularly cardiology procedures, involve accessing a corporeal vessel through a percutaneous sheath. The sheath necessarily requires the formation of a hole or opening in the vessel wall so that a medical procedure can be performed via the sheath. After the particular medical procedure has been performed, the sheath must eventually be removed from the vessel and the access hole in the vessel wall closed.

A number of prior vascular closure devices have been developed in attempting to provide a solution for the problem of closing a hole in the vessel wall. Tissue approximation typically involves passing a length of suture into and through adjacent vessel and subcutaneous tissue, across the vessel opening, and back into and through adjacent vessel and subcutaneous tissue. Certain prior closure devices have involved relatively complicated methods and devices for extracting a length of suture from inside the vessel so that the physician can approximate tissue surrounding the hole in the vessel wall through use of the suture.

Closing large bore holes in a vessel wall may pose additional challenges. Large bore holes are typically greater than 10 French (F) in size, and more often in the range of at least 15-20 F. The size of a large bore hole creates the possibility of greater blood loss in less amount of time as compared to small bore holes. Further, closing a large bore hole often requires multiple sutures, the failure of any of which during closure of the hole may result in significant blood loss and even a complete blow out of the hole. In at least some cases, a complete blow out may put a patient's life at risk.

Document US2002188312 A1 describes a catheter having an elongated tubular member having a proximal and a distal end. A balloon is positioned at the distal end of the tubular member. A guidewire tubular member extends from a position proximal the balloon through the balloon to a position distal the balloon, the guidewire tubular member has a proximal segment having a first flexibility and a distal segment having a second flexibility. The distal segment is more flexible than the proximal segment. A bond joins the balloon distal end to the proximal segment and the distal segment.

Further balloon catheters are described in documents US 2011282195, EP 1479409 A1, US 2003167038 A1, US2007016133 A1, US 2006064074 A1, US 2013006174 A1, WO 2012162651 A1, US2008171979 A1, US 2008077085 A1.

Opportunities exist for improving procedures and related devices for closing large bore holes and limiting the risk of significant blood loss and complete blow out of the large bore hole during a closure procedure.

### SUMMARY

One aspect of the present disclosure relates to a blood flow cessation device that includes a catheter, an expandable member, and a rapid exchange port. The catheter includes a distal end portion and a proximal end portion. The expandable member is positioned at the distal end portion and is selectively expandable to stop blood flow through a vessel. The rapid exchange port is formed in the catheter at a location proximal of the expandable member and is configured to receive a guidewire that passes through the distal end portion of the catheter to be positioned within the vessel.

The blood flow cessation device may also include a guidewire lumen that extends from the rapid exchange port distally through the distal end portion of the catheter. The expandable member may include an inflatable balloon, and the catheter may include an inflation lumen that extends from the proximal end portion to the expandable member. The expandable member may include a shape memory material. The expandable member may include a Nitinol mesh positioned in an elastic membrane. The blood flow cessation device may include a valve positioned at the proximal end portion that is operable to control flow of inflation fluid relative to the inflation lumen. The valve may include one of a duckbill valve and a Tuohy Borst valve. The blood flow cessation device may include a manifold positioned at the proximal end portion that is sized to pass through an introducer sheath. The rapid exchange port may be formed in a sidewall of the catheter.

The present invention relates to a blood flow cessation system that includes a blood flow cessation device, a treatment device, a guidewire and an insertion sheath. The blood flow cessation device includes a catheter having a distal end portion and a proximal end portion, an expandable member positioned at the distal end portion, and a rapid exchange port formed in the catheter at a location proximal of the expandable member and distal of the proximal end portion. The catheter includes an inflation lumen extending from the proximal end portion to the distal end portion. The expandable member comprises an inflatable balloon positioned at the distal end portion and being selectively expandable to stop blood flow through a vessel. The rapid exchange port is formed in a sidewall of the catheter. A structure defines a guidewire lumen extending from the rapid exchange port distally through the distal end portion of the catheter, with the expandable member having a proximal portion connected to the distal end portion of the catheter and a distal portion connected to a distal end of the structure defining the guidewire lumen, with the structure defining the guidewire lumen extending through the inflation lumen such that inflation fluid flowing therethrough to inflate the inflatable balloon flows around the guidewire lumen. The guidewire extends through the rapid exchange port and through the distal end portion of the catheter. The blood flow cessation device is configured to advance along the guidewire through a vessel puncture and into a vessel where the expandable member is expandable to limit blood flow in the vessel. The treatment device comprises a large bore closure device and is configured to advance along the guidewire to the vessel puncture. The insertion sheath is positionable in the vessel puncture. The blood flow cessation device is insertable through the insertion sheath into the vessel.

A further aspect of the present disclosure relates to a method of treating a vessel having a vessel puncture. The method includes providing a blood flow cessation device, a guidewire, and a treatment device. The blood flow cessation device includes a catheter having distal and proximal end portions, an expandable member, and a rapid exchange port positioned proximal of the expandable member and distal of the proximal end portion. The method includes advancing the guidewire through the vessel puncture and into the vessel, advancing the blood flow cessation device along the guidewire and into the vessel with the guidewire extending through the rapid exchange port, operating the expandable member to limit blood flow through the vessel, advancing the treatment device along the guidewire through the vessel puncture, and treating the vessel with the treatment device.

The method may also include providing an insertion sheath, and inserting the insertion sheath through the vessel puncture, wherein the guidewire extends through the insertion sheath. Advancing the blood flow cessation device may include advancing the blood flow cessation device through the insertion sheath. The method may include withdrawing the insertion sheath proximally along the blood flow cessation device prior to advancing the treatment device along the guidewire. Operating the expandable member may include delivering a volume of inflation fluid through the catheter to expand the expandable member. Treating the vessel may include at least partially closing the vessel puncture.

The foregoing and other features, utilities, and advantages of the invention will be apparent from the following detailed description of the invention with reference to the accompanying drawings.

### BRIEF DESCRIPTION OF THE DRAWINGS

FIG. 1A is a side view of an example blood flow cessation device in accordance with the present disclosure.
FIG. 1B is a cross sectional view of a distal end portion of the blood flow cessation device of FIG. 1A.
FIGS. 2A-2H show steps of sealing a vessel puncture using a vascular closure system, which includes the blood flow cessation device of FIG. 1A.
FIGS. 3A and 3B show an example occlusion member for use with the blood flow cessation device of FIG. 1A.
FIG. 4 shows an example manifold and valve for use at a proximal end of the blood flow cessation device at FIG. 1A.

### DETAILED DESCRIPTION

The present disclosure is generally directed to systems and methods for closing large bore vessel punctures. One aspect of the present disclosure is directed to a blood flow cessation device that provides a mechanical apparatus for temporarily stopping the flow of blood in a vessel (*e*.*g*., femoral artery or a vein) during a procedure where there is a risk of blood loss that could necessitate a transfusion or other adverse event. The blood flow cessation device may be an accessory device that travels over a guidewire, through the vessel puncture, and into the vessel interior. The blood flow cessation device may include an expandable portion that occludes blood flow through the vessel thereby limiting the volume of blood that is available to pass through the vessel puncture. The blood flow cessation device may include a rapid exchange port that allows insertion of devices proximal to the occlusion location. These devices may include, for example, different sheaths that may be involved in a sheath exchange, a large bore closure device, or other devices associated with closing the vessel puncture.

The blood flow cessation device may be referred to as a rapid exchange device. The blood flow cessation device may include a proximal shaft, a distal shaft, and a guidewire port located at a junction between the proximal and distal shafts. The distal shaft may include an expandable member such as a balloon. The expandable member may be expanded using an inflation fluid. Alternatively, the expandable member may comprise a mechanical expansion device such as a Nitinol disk, coil member, or other expandable feature. The expandable portion may be positioned (*e*.*g*., disposed) at a distal most end of the blood flow cessation device at a location distal of the guidewire port. The expandable member is operable within the vessel to stop blood flow in at least one direction through the blood vessel thereby limiting blood flow through the vessel puncture. The vessel puncture may be referred to as an access site, or, more specifically, a large bore access site.

The blood flow cessation device may include a proximal manifold having a valve. The valve may control fluid flow to the expandable member. The manifold may have a low profile construction of a size that permits a sheath to traverse over the manifold. In at least one example, a sheath is removed over the manifold or advanced over the manifold as desired while the valve of the manifold maintains the expandable member in an expanded state to maintain occlusion of blood flow through the vessel.

In at least some examples, the blood flow cessation device and associated proximal manifold may be constructed to permit a sheath exchange from a small bore sheath *(e.g.,* 1 F to about 10 F) to a large bore sheath *(e.g.,* greater than 10 F *(e.g.,* 11 F to 25 F)). In one example, after the large bore sheath has been placed, the blood flow cessation device may be removed from the vessel and later inserted and used again after the remainder of the procedure has been completed and the large bore sheath is to be removed. The blood flow cessation device may have separate utility when used with a sheath that needs to be removed and the puncture site closed a small amount, at which time the guidewire and blood flow cessation device may be removed in a single step.

The example blood flow cessation devices and related vascular closure systems disclosed herein may provide for active control of blood loss from the same vessel puncture (*e*.*g*., access site) by which access into the vessel is provided for treating the patient. The blood flow cessation device may remain positioned within the vessel, whether in an expanded or unexpanded state, to occlude blood flow during various steps of treating a patient or closing the vessel puncture through which the blood flow cessation device extends. The blood flow cessation device may be expanded relatively quickly and at any time to occlude blood flow, thereby limiting the risk of significant blood loss and possible blood transfusion, such as in the case of an unexpected complete loss of closure of the vessel puncture. The blood flow cessation device and associated vascular closure systems disclosed herein may eliminate the need to rely solely on application of pressure to the vessel upstream of the access site and quick device exchanges to avoid significant blood loss through the access site.

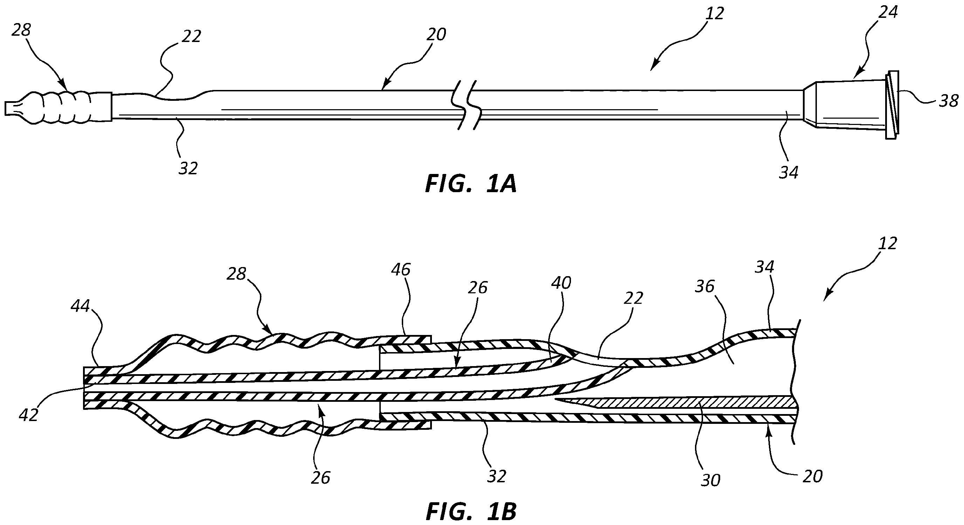

Referring now to FIGS. 1A and 1B, an example blood flow cessation device 12 is shown including a shaft 20, a rapid exchange port 22, a manifold 24, a wire lumen 26, an occlusion member 28, and a core wire 30. The shaft 20 includes distal and proximal end portions 32, 34. The rapid exchange port 22 is typically positioned at a juncture between the distal and proximal end portions 32, 34. The shaft 20 defines a lumen 36 that extends from the proximal end portion 34 to the occlusion member 28 at the distal end portion 32.

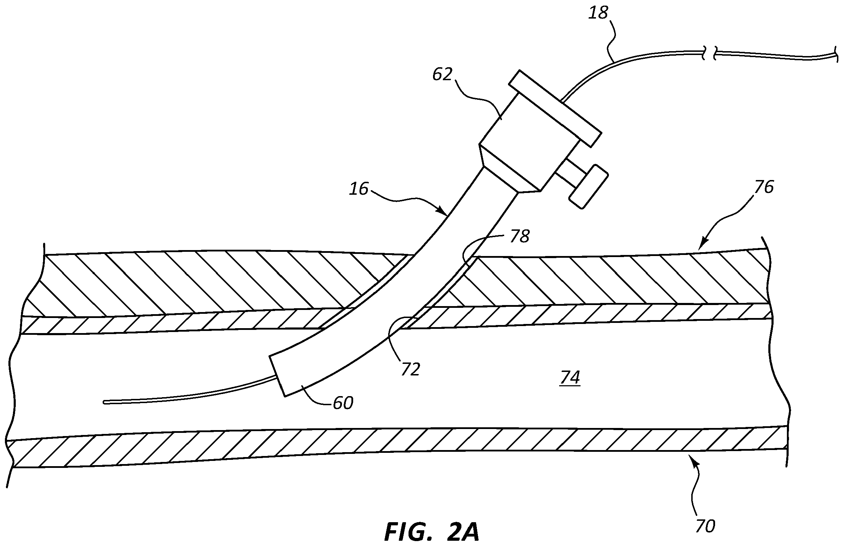

The wire lumen 26 extends from the rapid exchange port 22 distally to a distal waist 44 of the occlusion member 28. The wire lumen 26 may define a pathway for a guidewire 18 (*see* FIGS. 2A-2H) to extend through the distal end portion 32 of shaft 20 and through the occlusion member 28. In one example, inflation fluid flows through the lumen 36 of shaft 20, around the wire lumen 26, and into the occlusion member 28 to inflate the occlusion member 28.

The occlusion member 28 may include distal and proximal waists 44, 46. The proximal waist 46 is connected to the distal end portion 32 of shaft 20. The distal waist 44 is connected to a distal end 42 of a structure defining the wire lumen 26. A proximal end 40 of the wire lumen 26 is attached to the shaft 20 at the rapid exchange port 22.

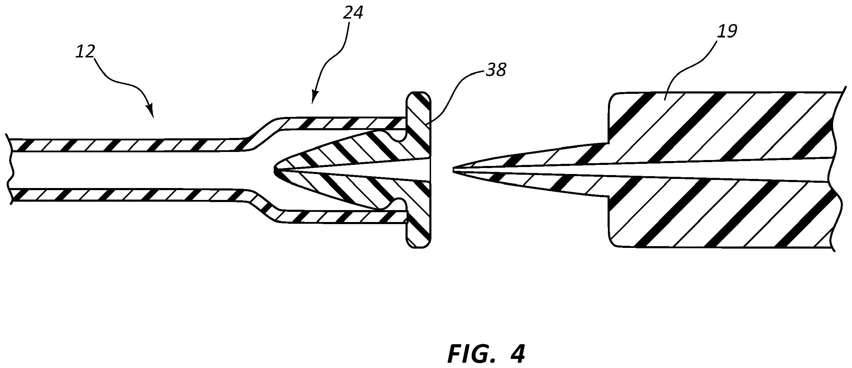

The manifold 24 may include a valve 38. FIG. 4 shows a duck bill valve as an example valve in the manifold 24. The duckbill valve 38 may receive an inflation member 19 that provides a source of inflation fluid. The inflation member 19 may extend through the valve 38 and provide a sealed interface therewith while delivering inflation fluid to expand occlusion member 28. After the inflation member 19 is removed from valve 38, the valve 38 may maintain a fluid pressure within the blood flow cessation device 12 to maintain the occlusion member 28 in an expanded state. Maintaining the occlusion member 28 in an expanded state after being detached from the inflation member 19 may be useful when conducting a sheath exchange over the manifold 24.

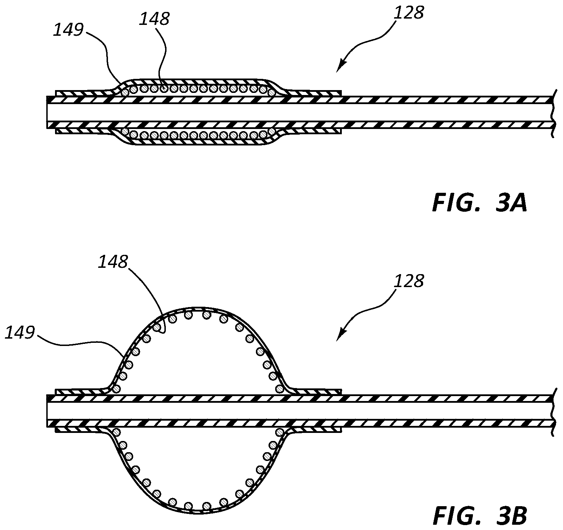

The occlusion member 28 may have other configurations besides being an inflation member. For example, FIGS. 3A and 3B show an alternative occlusion member 128 that includes an expansion mesh 148 covered by a membrane 149. The expansion mesh 148 may be activated from a low profile, unexpanded position as shown in FIG. 3A, to an expanded, enlarged position as shown in FIG. 3B. The expansion mesh 148 may be activated using, for example, a pull wire, electric stimulation, or application of a rotation force. The expansion mesh 148 may be configured as a coil such as a helical coil, or other mechanical structure.

The blood flow cessation device 12 may be part of a vascular closure system that also includes a vascular closure device 14, a sheath 16, and a guidewire 18. An example vascular closure system is described with reference to FIGS. 2A-2H as part of a method of sealing a vessel puncture 72 in a vessel 70. The vessel 70 includes a vessel interior 74. The vessel puncture 72 is accessible through a percutaneous incision 78 of a tissue layer 76.

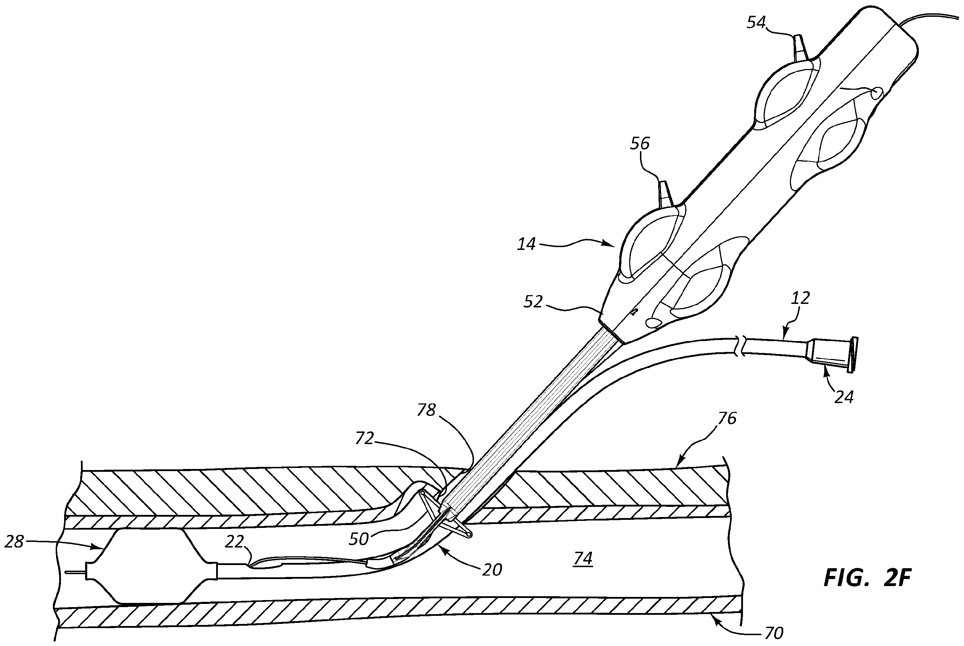

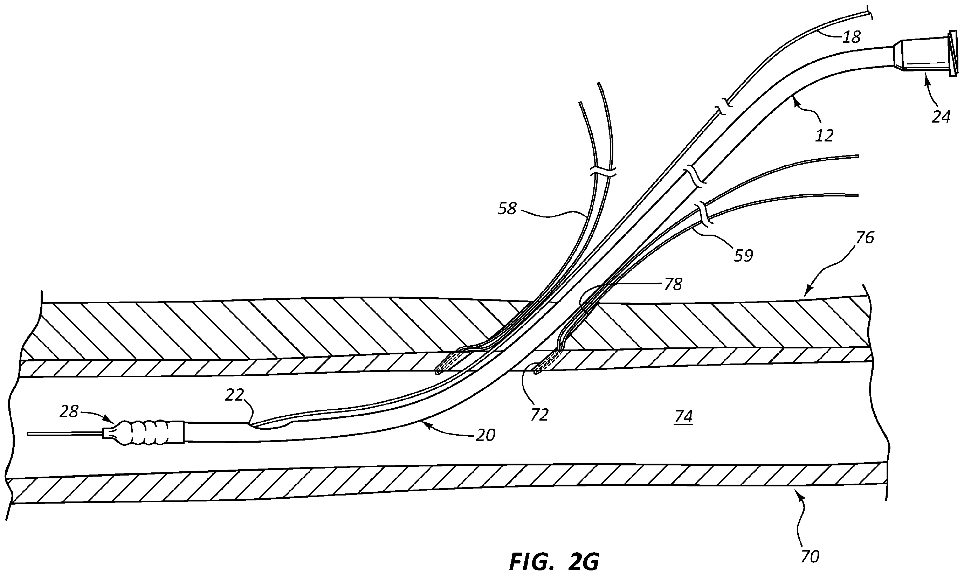

FIG. 2F shows the vascular closure device 14 including an anchor 50, a handle 52, a needle actuator 54, an anchor actuator 56, and first and second sutures 58, 59 (*see* FIG. 2G). The vascular closure device 14 may also include at least two needles (not shown) that are advanced through the vessel wall to position the first and second sutures 58, 59 extending through the vessel wall upon actuation of the needle actuator 54. Other types of vascular closure devices may be used in association with a blood flow cessation device 12 to assist in closing the vessel puncture 72 prior to, during, or after occluding blood flow through the vessel interior 74 using the blood flow cessation device 12.

The sheath 16 may include a distal end 60 and a hub 62 as shown in FIG. 2A. The sheath 16 may provide access to the vessel interior 74 as part of treating the vessel using a treatment device (not shown). An initial step in sealing the vessel puncture 72 may include advancing the guidewire 18 through the sheath 16 and into the vessel interior 74. The sheath 16 may be prepositioned extending through the vessel puncture 72 to position the distal end 60 within the vessel interior 74. In other arrangements, the guidewire 18 is prepositioned extending through the vessel puncture 72, and the sheath 16 is advanced over the guidewire 18 into the inserted position shown in FIG. 2A.

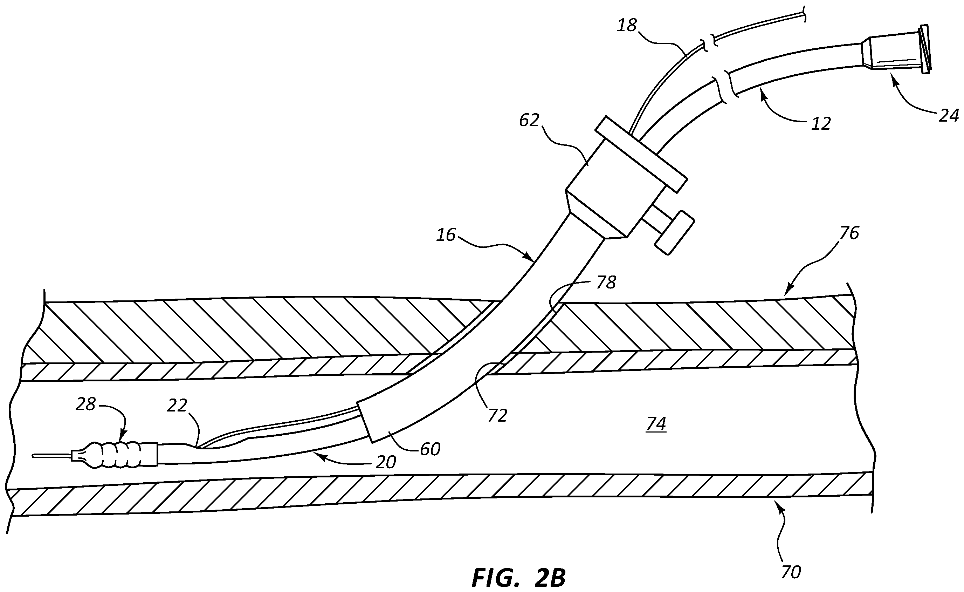

With the guidewire 18 positioned extending through the vessel puncture 72 and into the vessel interior 74, a proximal end of the guidewire 18 is fed through the wire lumen 26 of the blood flow cessation device 12 and out through the rapid exchange port 22. The blood flow cessation device 12 is then advanced over the guidewire 18 and through the sheath 16 until the occlusion member 28 is positioned within the vessel interior 74 at a location distal of the distal end 60 of the sheath 16, as shown in FIG. 2B.

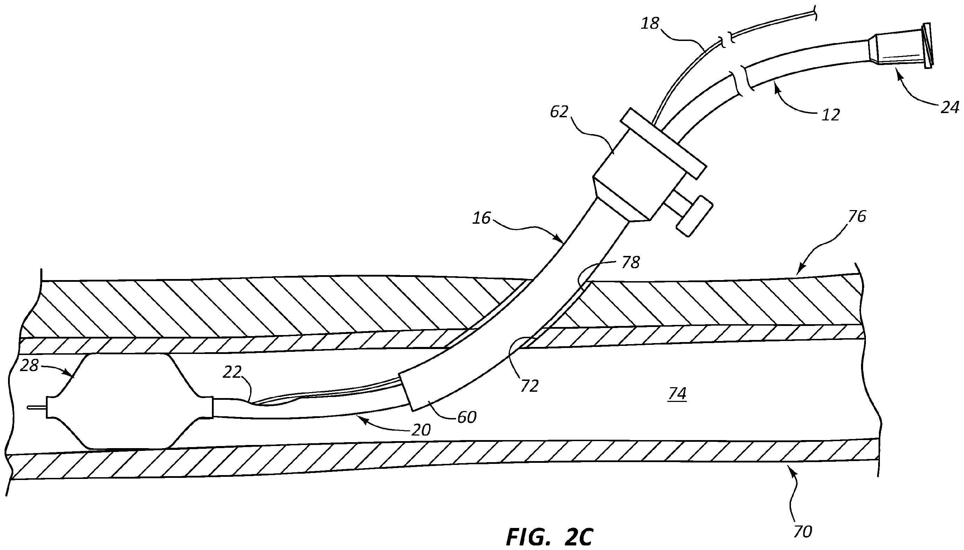

Referring to FIG. 2C, the occlusion member 28 is expanded to occlude blood flow through the vessel interior 74. As discussed above, the occlusion member 28 may be expanded by delivering a volume of inflation fluid through the lumen 36 of shaft 20 from an inflation member 19. The flow of inflation fluid is controlled at the proximal end by the valve 38 of manifold 24. Alternatively, the occlusion member 28 may be expanded using the expansion mesh 148 shown in FIGS. 3A and 3B or some other selectively expandable device.

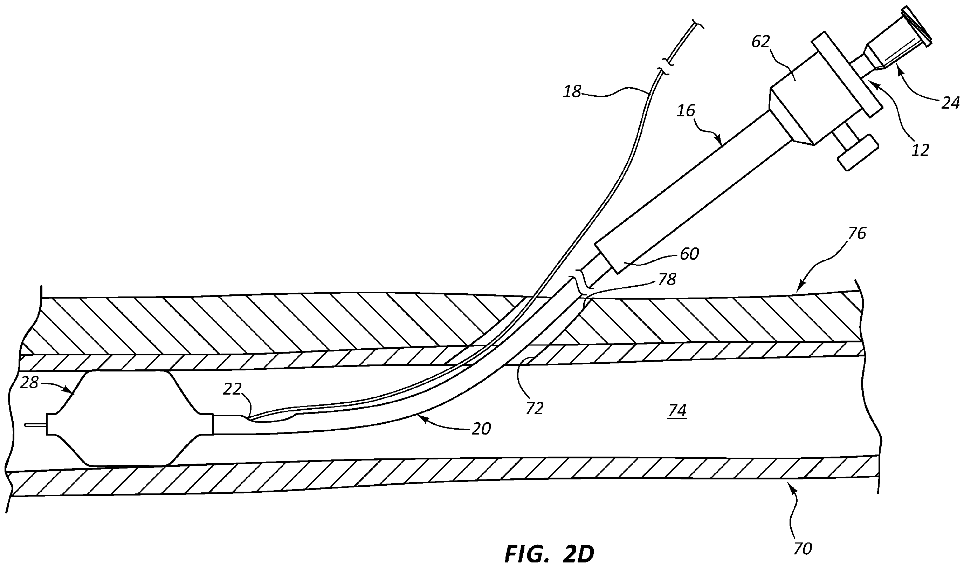

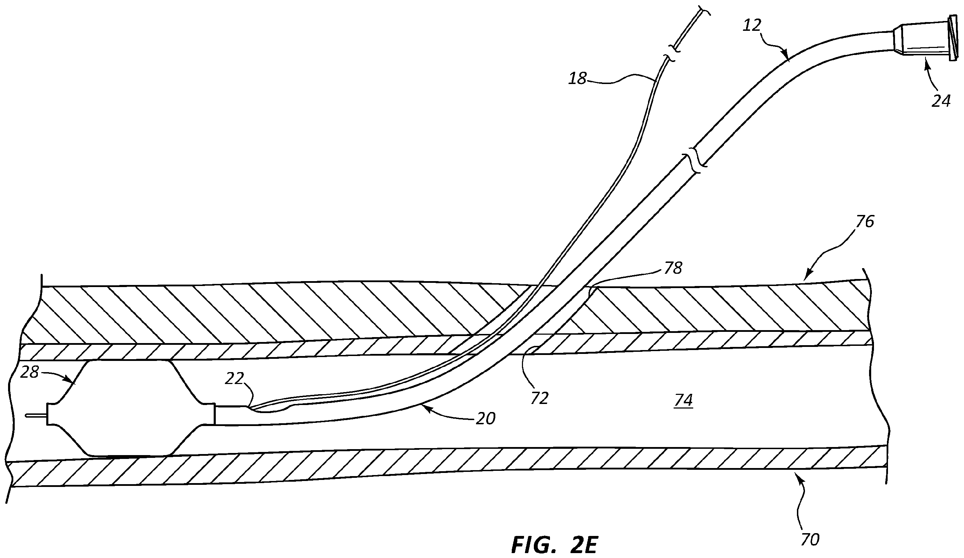

After expansion of the occlusion member 28 and occlusion of blood flow through the vessel interior 74, the sheath 16 may be removed from the vessel puncture 72 as shown in FIG. 2D. The blood flow cessation device 12 may have a sufficient length to provide positioning of the sheath 16 removed from the vessel puncture 72 and percutaneous incision 78 without being removed over the manifold 24, as shown in FIG. 2D. Alternatively, as shown in FIG. 2E, the sheath 16 may be completely removed from the blood flow cessation device 12 by being withdrawn over the relatively low profile manifold 24. Whether using the arrangement at FIG. 2D or the arrangement at FIG. 2E, the guidewire 18 is made accessible outside of the sheath 16 for use in directing the vascular closure device 14 to the vessel puncture 72.

FIG. 2F shows the vascular closure device 14 advanced over the guidewire 18 and extending through the vessel puncture 72. The anchor 50 is operated from a retracted position to an expanded position by operation of the anchor actuator 56, as shown in FIG. 2F. The needle actuator 54 is then operated to advance needles (not shown) through the vessel wall to position first and second sutures 58, 59 extending through the vessel wall, as shown in FIG. 2G. The vascular closure device 14 is then withdrawn from the tissue puncture to leave behind the first and second sutures 58, 59 extending across the vessel puncture 72. Further details concerning operation of a vascular closure device that places sutures across a vessel puncture are provided in U.S. Patent Application No. 61/494,345, filed on June7, 2011, and entitled "Large Bore Closure Device and Methods," which is referenced herein.

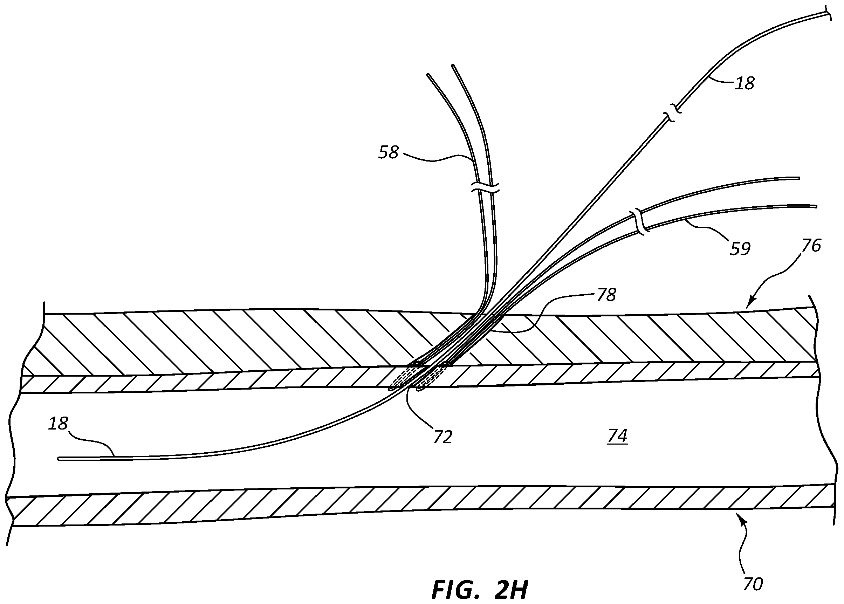

At least one of the first and second sutures 58, 59 may be at least partially tightened to begin closing the vessel puncture 72 while the blood flow cessation device 12 extends through the vessel puncture 72. When the operator confirms that the vessel puncture 72 may be adequately sealed using the first and second sutures 58, 59, the operator collapses the occlusion member 28 as shown in FIG. 2G and withdraws blood flow cessation device 12 from the vessel puncture 72 as shown in FIG. 2H. The operator may further cinch and/or tie off at least one of the first and second sutures 58, 59 to seal the vessel puncture 72 as shown in FIG. 2H. The guidewire 18 may be withdrawn prior to, during, or after tying off or one or both of the first and second sutures 58, 59. In some examples, the guidewire 18 may be withdrawn concurrently with the blood flow cessation device 12. In further examples, the guidewire 18, blood flow cessation device 12 and vascular closure device 14 may all be withdrawn concurrently from the vessel puncture 72. In still further examples, the guidewire 18 may be withdrawn prior to withdrawing one or both of the blood flow cessation device 12 and vascular closure device 14.

The blood flow cessation device 12 is shown in the examples herein having a single occlusion member 28. The occlusion member 28 may provide sealing contact with interior surfaces of the vessel 70 at a single axial location along the length of the vessel interior 74. Many other example constructions are possible for the blood flow cessation device. In one example, the occlusion member 28 includes a plurality of spaced apart expanded portions that each provides a separate sealing contact with an inner surface of the vessel 70 at different spaced apart locations along a length of the vessel interior 74. In other examples, the blood flow cessation device includes a plurality of separate occlusion members. The plurality of occlusion members may be separately expandable or may concurrently expand upon delivery of, for example, an inflation fluid to all of the occlusion members.

In other examples, the blood flow cessation device includes a plurality of rapid exchange ports and associated wire lumens to accommodate multiple guidewires. In other examples, the rapid exchange port 22 and wire lumen 26 are sized to accommodate a plurality of guidewires.

The shaft 20 shown in the figures includes a single lumen 36. In other examples, the shaft 20 may include a plurality of lumens, such as a plurality of coaxially arranged lumens or a plurality of lumens arranged side-by-side in a multi-lumen shaft construction.

A method of treating a vessel having a vessel puncture may be accomplished using the blood flow cessation device and associated vascular closure system described herein. The method may include providing a blood flow cessation device, a guidewire, and a treatment device. The blood flow cessation device may comprise a catheter or tube such as the shaft 20 describe above. The catheter may include distal and proximal end portions. The blood flow cessation device may also include an expandable member and a rapid exchange port positioned proximal of the expandable member and distal of the proximal end portion.

The method may include advancing the guidewire through the vessel puncture and into the vessel and then advancing the blood flow cessation device along the guidewire and into the vessel with the guidewire extending through the rapid exchange port. The expandable member may be operated to expand within the vessel to limit blood flow through the vessel, thereby limiting the amount of blood available to flow through the vessel puncture. The treatment device is then advanced along the guidewire through the same vessel puncture that is used to access the vessel. Treating a vessel may include closing the vessel puncture. Closing the vessel puncture may include positioning at least one suture extending across the vessel puncture or positioning a sealing member adjacent to the vessel puncture.

The method may also include providing an insertion sheath that is inserted through the vessel puncture, and the wire extends through the insertion sheath prior to advancing the blood flow cessation device along a guidewire and into the vessel. Expanding the expandable member may include delivering a volume of inflation fluid to the expandable member. The method may also include withdrawing the insertion sheath over the blood flow cessation device prior to advancing a treatment device along the guidewire and into the vessel puncture. The insertion sheath may be removed completely off a proximal end of the blood flow cessation device such as, for example, removal over a manifold having a valve that is positioned at a proximal end of the blood flow cessation device.

As used in this specification and the appended claims, the terms "engage" and "engageable" are used broadly to mean interlock, mesh, or contact between two structures or devices. Likewise "disengage" or "disengageable" means to remove or capable of being removed from interlock, mesh, or contact. A "tube" is an elongated device with a passageway. The passageway may be enclosed or open *(e.g.,* a trough). A "lumen" refers to any open space or cavity in a bodily organ, especially in a blood vessel. The words "including" and "having," as well as their derivatives, as used in the specification, including the claims, have the same meaning as the word "comprising."

The preceding description has been presented only to illustrate and describe exemplary embodiments of the invention. It is not intended to be exhaustive or to limit the invention to any precise form disclosed. Many modifications and variations are possible in light of the above teaching. It is intended that the scope of the invention be defined by the following claims.

## Claims

1. A blood flow cessation system, comprising:
a blood flow cessation device (12) comprising:
a catheter (20) having a distal end portion (32), a proximal end portion (34), and an inflation lumen (36) extending from the proximal end portion (34) to the distal end portion (32);
an expandable member (28) comprising an inflatable balloon positioned at the distal end portion (32) and being selectively expandable to stop blood flow through a vessel;
a rapid exchange port (22) formed in a sidewall of the catheter (20) at a location proximal of the expandable member (28); and
a structure defining a guidewire lumen (26) extending from the rapid exchange port (22) distally through the distal end portion (32) of the catheter (20), with the expandable member (28) having a proximal portion (46) connected to the distal end portion (32) of the catheter (20) and a distal portion (44) connected to a distal end (42) of the structure defining the guidewire lumen (26), with the structure defining the guidewire lumen (26) extending through the inflation lumen (36) such that inflation fluid flowing therethrough to inflate the inflatable balloon flows around the guidewire lumen (26); and
a treatment device (14);
a guidewire (18) extending through the rapid exchange port (22) and through the distal end portion (32) of the catheter (20);
wherein the blood flow cessation device (12) is configured to advance along the guidewire (18) through a vessel puncture and into a vessel where the expandable member (28) is expandable to limit blood flow in the vessel,
**characterized in that** the treatment device (14) comprises a large bore closure device, and the treatment device (14) is configured to advance along the guidewire (18) to the vessel puncture, and the blood flow cessation system further comprises an insertion sheath (16) positionable in the vessel puncture, the blood flow cessation device (12) being insertable through the insertion sheath (16) into the vessel, with the treatment device (14) being positioned on the guidewire (18) external to the insertion sheath (16).

2. The blood flow cessation system of claim 1, wherein the blood flow cessation device further comprises a valve (38) positioned at the proximal end portion (34) and operable to control flow of inflation fluid relative to the inflation lumen (36).

3. The blood flow cessation system of claim 2, wherein the valve (38) comprises one of a duckbill valve and a Tuohy Borst valve.

4. The blood flow cessation system of claim 1, wherein the blood flow cessation device further comprises a manifold (24) positioned at the proximal end portion (34), the manifold (24) being sized to pass through an introducer sheath.

## Patentansprüche

1. Blutflussunterbrechungssystem, umfassend:
eine Blutflussunterbrechungsvorrichtung (12), umfassend:
einen Katheter (20) mit einem distalen Endstück (32), einem proximalen Endstück (34) und einem Inflationslumen (36), das sich von dem proximalen Endstück (34) zu dem distalen Endstück (32) erstreckt;
ein expandierbares Element (28), das einen füllbaren Ballon umfasst, der an dem distalen Endstück (32) positioniert ist und das selektiv expandierbar ist, um den Blutfluss durch ein Gefäß zu unterbrechen;
einen Schnellwechselanschluss (22), der in einer Seitenwand des Katheters (20) an einer Position proximal zu dem expandierbaren Element (28) ausgebildet ist; und
eine Struktur, die ein Führungsdrahtlumen (26) definiert, das sich von dem Schnellwechselanschluss (22) distal durch das distale Endstück (32) des Katheters (20) erstreckt, wobei das expandierbare Element (28) einen proximalen Teil (46) aufweist, der mit dem distalen Endstück (32) des Katheters (20) verbunden ist, und einen distalen Teil (44), der mit einem distalen Ende (42) der das Führungsdrahtlumen (26) definierenden Struktur verbunden ist, wobei sich die das Führungsdrahtlumen (26) definierende Struktur durch das Inflationslumen (36) erstreckt, so dass dort hindurch fließendes Inflationsfluid, um den füllbaren Ballon zu füllen, um das Führungsdrahtlumen (26) fließt; und
eine Behandlungsvorrichtung (14);
einen Führungsdraht (18), der sich durch den Schnellwechselanschluss (22) und durch das distale Endstück (32) des Katheters (20) erstreckt;
wobei die Blutflussunterbrechungsvorrichtung (12) so gestaltet ist, dass sie entlang dem Führungsdraht (18) durch eine Gefäßpunktur und in ein Gefäß treten kann, wo das expandierbare Element (28) expandierbar ist, um den Blutfluss in dem Gefäß einzuschränken,
**dadurch gekennzeichnet, dass** die Behandlungsvorrichtung (14) eine Large-Bore-Verschlussvorrichtung umfasst, und wobei die Behandlungsvorrichtung (14) so gestaltet ist, dass sie entlang dem Führungsdraht (18) zu der Gefäßpunktur verlaufen kann, und wobei das Blutflussunterbrechungssystem ferner eine Einführschleuse (16) umfasst, die in der Gefäßpunktur positionierbar ist, wobei die Blutflussunterbrechungsvorrichtung (12) durch die Einführschleuse (16) in das Gefäß einführbar ist, wobei die Behandlungsvorrichtung (14) außerhalb der Einführschleuse (16) an dem Führungsdraht (18) positioniert ist.

2. Blutflussunterbrechungssystem nach Anspruch 1, wobei die Blutflussunterbrechungsvorrichtung ein Ventil (38) umfasst, das an dem proximalen Endstück (34) positioniert und so funktionsfähig ist, dass es den Fluss von Inflationsfluid im Verhältnis zu dem Inflationslumen (36) regelt.

3. Blutflussunterbrechungssystem nach Anspruch 2, wobei das Ventil (38) ein Entenschnabelventil oder ein Tuohy-Borst-Ventil umfasst.

4. Blutflussunterbrechungssystem nach Anspruch 1, wobei die Blutflussunterbrechungsvorrichtung ferner ein Übergangsstück (24) umfasst, das an dem proximalen Endstück (34) positioniert ist, wobei das Übergangsstück (24) so bemessen ist, dass es durch eine Einführschleuse verläuft.

## Revendications

1. Système de cessation de circulation sanguine, comprenant :
un dispositif de cessation de circulation sanguine (12) comprenant :
un cathéter (20) ayant une partie d'extrémité distale (32), une partie d'extrémité proximale (34), et une lumière de gonflage (36) s'étendant de la partie d'extrémité proximale (34) à la partie d'extrémité distale (32) ;
un élément expansible (28) comprenant un ballonnet gonflable positionné au niveau de la partie d'extrémité distale (32) et étant expansible sélectivement pour arrêter la circulation sanguine à travers un vaisseau ;
un orifice d'échange rapide (22) formé dans une paroi latérale du cathéter (20) à un emplacement proche de l'élément expansible (28) ; et
une structure définissant une lumière de fil-guide (26) s'étendant à partir de l'orifice d'échange rapide (22) distalement à travers la partie d'extrémité distale (32) du cathéter (20), l'élément expansible (28) ayant une partie proximale (46) reliée à la partie d'extrémité distale (32) du cathéter (20) et une partie distale (44) reliée à une extrémité distale (42) de la structure définissant la lumière de fil-guide (26), la structure définissant la lumière de fil-guide (26) s'étendant à travers la lumière de gonflage (36) de sorte que le fluide de gonflage s'écoulant à travers celle-ci pour gonfler le ballonnet gonflable s'écoule autour de la lumière de fil-guide (26) ; et
un dispositif de traitement (14) ;
un fil-guide (18) s'étendant à travers l'orifice d'échange rapide (22) et à travers la partie d'extrémité distale (32) du cathéter (20) ;
le dispositif de cessation de circulation sanguine (12) étant conçu pour avancer le long du fil-guide (18) à travers une perforation de vaisseau et dans un vaisseau où l'élément expansible (28) est expansible pour limiter la circulation sanguine dans le vaisseau,
**caractérisé en ce que** le dispositif de traitement (14) comprend un dispositif de fermeture à grand alésage, et le dispositif de traitement (14) est conçu pour avancer le long du fil-guide (18) jusqu'à la perforation de vaisseau, et le système de cessation de circulation sanguine comprend en outre une gaine d'insertion (16) pouvant être positionnée dans la perforation de vaisseau, le dispositif de cessation de circulation sanguine (12) pouvant être inséré par la gaine d'insertion (16) dans le vaisseau, le dispositif de traitement (14) étant placé sur le fil-guide (18) à l'extérieur de la gaine d'insertion (16).

2. Système de cessation de circulation sanguine selon la revendication 1, le dispositif de cessation de circulation sanguine comprenant en outre une valve (38) positionnée au niveau de la partie d'extrémité proximale (34) et permettant de réguler le flux de fluide de gonflage par rapport à la lumière de gonflage (36).

3. Système de cessation de circulation sanguine selon la revendication 2, la valve (38) comprenant une valve à bec de canard ou une valve de Tuohy Borst.

4. Système de cessation de circulation sanguine selon la revendication 1, le dispositif de cessation de circulation sanguine comprenant en outre un collecteur (24) positionné au niveau de la partie d'extrémité proximale (34), le collecteur (24) étant dimensionné pour passer à travers une gaine d'introduction.
